# EUROPEAN PATENT APPLICATION

(11) **EP 3 854 375 A1**
(43) Date of publication of application: **28.07.2021**
(21) Application number: 21152239.6
(22) Date of filing: 19.01.2021
(51) Int. Cl.: A61K 8/34, A61K 8/73, A61Q 19/08, A61K 8/02

(54) **MICRO-SPICULE COMPOSITION TO CONTROL ITS SHAPE AND METHOD FOR PRODUCING THE SAME**

(30) Priority: 23.01.2020 KR 20200009346; 08.04.2020 US 202016843235
(71) Applicant: Paean Aesthetics Inc, Daejeon 34013 (KR)
(72) Inventor: HAN, Kyuboem, 34028 Daejeon (KR); HA, JongCheon, 34048 Daejeon (KR)
(74) Representative: Brann AB

(57) **Abstract**

The present invention relates to a composition for controlling the shape of the micro-spicule particles, micro-spicule particles molded by water-soluble materials, and its method for producing the micro-spicule particles molding with water-soluble materials having a sharp pointed vertex without tip collapse by controlling the amount of the alkylated glycerin compound in the solution loaded onto the multiple pattern engraved mold. The shape of molded micro-spicule s can be controlled by using alkylated glycerin and thus various types of water-soluble micro-spicule particles can be prepared when mixed with a non-aqueous formulation and applied to the skin, the water-soluble micro-spicule particles prepared by the present invention provide advantageous scrubbing effect.

## Description

### BACKGROUND OF THE INVENTION

### 1. Field of the Invention

The present invention relates to a micro-spicule for promoting the absorption of a cosmetic material into the skin. More particularly the present invention relates to a composition for controlling the shape of micro-spicule particles molded by water-soluble substances and a method for producing the same.

More specifically, the present invention is a micro-spicule particle produced by molding water-soluble materials comprising an alkylated glycerin compounds and hyaluronic acid in a multiple pattern engraved mold, wherein the water-soluble micro-spicule particles are present in the content of the alkylated glycerin compounds. Therefore, the present invention relates to a composition whose shape can be controlled within same engraved mold.

### 2. Description of the Prior Art

The delivery system of useful materials such as skin care or cosmetic materials or drugs into the body by coating the skin with the materials or applying a pack or patch to the skin enjoys the advantage of allowing the continuous transport of the materials, generating no pain, and being convenient for use.

However, because the stratum corneum, which is the outermost epidermal layer of skin, is 10 µm to 60 µm thick, it serves as a barrier to the penetration of foreign matter into the body, thus a delivery system using coating or patches is very poor in absorption efficiency.

Particularly, when the materials to be delivered are hydrophilic or have a large molecular weight, their absorption into the body becomes poorer.

Injection is an effective method to deliver a useful material into the body. Having dimensions of millimeters for diameter and centimeters for length, however, an injection needle stimulates many pain receptors, causing a significant pain in a subject to which the injection needle is applied. In addition, injection should be performed in hospitals or specialized skin care institutes and cannot be easily used at home.

To overcome the drawback of conventional injection, micro-needles were developed to have a diameter of tens to hundreds µm and a length of hundreds to thousands µm. The micro-needles form micropores in the stratum corneum so that they allow materials, even though hydrophilic or large, to be readily absorbed into the skin or delivered into the body through the skin.

In addition, when applied to the site that is pierced with the conventional micro-needles, useful materials are absorbed or delivered into the body at better rates. Further, since micro-needles are too short to reach the dermis layer where nerve cells are distributed, no pain is generated upon the application of micro-needles. Even though micro-needles pierce into the dermis layer, they are smaller in diameter and length and stimulate fewer pain receptors than conventional injection needles so that the subject feels only minor pain.

MTS (Micro-spicule Therapy System) is one of the transdermal delivery systems developed to increase the absorption rate of active ingredients by damaging the stratum corneum.

Since conventional method is difficult to remove the air when filling the water-soluble material in the mold in which the small-sized polygonal pyramid shape multiple pattern is densely engraved for the production of water-soluble micro-spicule of various shapes from triangular pyramid to hexagonal pyramid and the water-soluble material is not completely filled into the mold, it is not easy to produce a sharp micro-spicule particles because it occurs.

In particular, when attempting to form a water-soluble micro-spicule by increasing the aspect ratio, which is the ratio between the diagonal length of the base of the polygonal pyramid and the height from the bottom to the pointed tip in order to obtain a sharp micro-spicule, many defects or incomplete defects in the water-soluble micro-spicule may occur due to the difficulty in releasing the micro-spicule particles.

WO 02/047555 discloses a device for treating the skin of a subject, including a head defining a convex head surface, and a plurality of spaced pins set in the head and protruding a predetermined distance from the head surface, and a method of treating the skin of a subject by pressing the device onto the skin of the subject with sufficient force for the pins to pierce the epidermis of the subject, leaving minute clefts in the epidermis.

U. S. Patent No. 5,487,726 describes a percutaneous vaccine applicator system adapted to apply a vaccine to the skin and to form minute clefts in the stratum corneum.

According to WO 02/047555 and U. S. Patent No. 5,487,726, however, it is inconvenient for the subject to use the device or the system because the dermal application of drugs and the percutaneous formation of minute clefts are conducted separately.

U. S. Patent No. 6,603,998 discloses a method for delivery molecules into cells by coating a micro-needle-type electrode with a molecule to be delivered and applying the electrode to the skin.

U. S. Patent No. 6,132,755 discloses a system for the actively controlled transcorneal delivery of a medicament, in which a drug container provided with micropins is designed to allow active substance to pass through capillary openings of the micropins into the subject with the aid of a pump.

U. S. Patent Application No 2005/0251088 discloses a method for the delivery of a medicament in which the medicament stored in pores of a porous needle is percutaneously delivered when the porous needle forms minute clefts in the epidermis.

U. S. Patent No. 3,964,482 discloses a drug delivery device, comprising a puncturing projection communicating with a drug reservoir wherein when the puncturing projection is applied to the stratum corneum of the epidermis, the drug is percutaneously administered into the skin by diffusion.

However, all of U. S. Patent Nos. 6,603,998, 6,132,755, and 3,964,482, and U. S. Patent Application No. 2005/0251088 require additional devices for the delivery of a material of interest.

An alternative method of facilitating the transdermal delivery of a skin care cosmetic material is to use a stratum corneum-abrading cosmetic before application of the skin care cosmetic to the skin. For this, the stratum corneum may be generally removed with a scrubbing agent.

The scrubbing agent must be washed off before a skin care cosmetic material is applied to the skin. Further, caution must be taken to avoid the entry of a scrubbing agent into the eye. If present in the eye, the scrubbing agent is difficult to remove. In addition to causing the sensation of foreign matter, the presence of scrubbing agent in the eye may abrade the corneum when the eye is rubbed.

Korean Patent No. 10-1206985 discloses a cosmetic composition for massaging the skin, comprising sugar scrubbers admixed with a non-aqueous cosmetic material. The cosmetic composition exhibits functions of moisturizing the skin, facilitating blood circulation, and removing dermal dead cells and corneal layers.

However, the sugar scrubbers of Korean Patent No. 10-1206985 have a general morphology of 8 or more faces, with blunt edges and large angles between the faces, and thus their scrubbing effect is not significant.

Leading to the present invention, intensive and thorough research of the present inventors into the delivery of skin care cosmetic substance or medicament resulted in the finding that tetrahedral or pyramidal micro-needles containing a dermal cosmetic material can be used as scrubbers to readily induce the percutaneous delivery of the dermal cosmetic material or the drug.

### SUMMARY OF THE INVENTION

The objective of the present invention is related to a molded micro-spicule particle for transdermal delivery and a method for producing the same, which maintains a sharp shape even after being separated from the mold.

The other objective of the present invention is related to the capability of adjusting the shape of water-soluble micro-spicule particles according to the content of the alkylated glycerin compounds without modifying the aspect ratio between the diagonal length of the bottom of the polygonal shape and the height from the bottom to the pointed tip and its method for producing the same.

Another object of the present invention is related to water-soluble micro-spicule particles with less porosity having a strength enough to be delivered transdermally and having adequate durability to maintain their shape before use and its method for producing the same.

### Technical Solution

The present invention can be achieved by adding alkylated glycerin compounds to the solution of a water-soluble carbohydrate compounds such as hyaluronic acid and dried for the production of micro-spicule particles, in order to produce molded water-soluble sharp and less fragile micro-spicule particles.

The present invention can make the shape of micro-spicule particles thick or slim by controlling the content of the alkylated glycerin compounds in the water-soluble micro-spicule particles manufacturing solution which is to be dried in the polygonal pyramidal engraved mold and can be achieved by providing a process comprising a composition ratio control process that can adjust the strength of the scrubbing particles.

In an example of the present invention, the alkylated glycerin compounds may be substituted with one or two hydroxyl groups of the three hydroxyl groups substituted or unsubstituted alkyl (C₄-C₂₀) oxy group.

In an example of the present invention, the water-soluble micro-spicule particles manufacturing solution for preparing the scrubbing particles may comprise 0.001 ∼ 1% by weight of the alkylated glycerin compounds.

The method for producing micro-spicule particles molded from a water-soluble material according to the present invention comprises the step of drying a mixture comprising hyaluronic acid, an alkylated glycerin compound and water.

The method for producing micro-spicule particles molded from a water-soluble material according to the present invention includes a mixing step of preparing a mixture comprising water, hyaluronic acid and an alkylated glycerin compound; A molding step of filling the mixture into a mold; and a drying step of drying the filled mixture.

The present invention may also provide a cosmetic composition comprising molded micro-spicule particles made with water-soluble material.

The composition for controlling the shape of the micro-spicule particles and the particles formed by the water-soluble material according to the present invention and a method for producing the same have a sharp shape without the end collapse as a pointed shape even after separation from the multiple pattern engraved mold, and also by producing the molded micro-spicule particles of various shapes to increase the scrub strength when scrubbing the skin, including in cosmetics or pharmaceutical compositions to maximize the skin delivery effect of the skin useful substances contained in cosmetics or pharmaceuticals.

In addition, micro-spicule particles according to the present invention shows the effect of having a strength that can be delivered trans-dermally while having less porosity and more durability enough to maintain the shape before use.

### BRIEF DESCRIPTION OF THE DRAWINGS

FIG. 1a is an image of pyramidal micro-spicule particles prepared without adding ethylhexyl glycerin to a water-soluble micro-spicule particle manufacturing solution.
FIG. 1b is an image of pyramidal-shaped micro-spicule particles prepared by adding 0.03% by weight of ethylhexylglycerin to a solution for preparing water-soluble micro-spicule particles.
FIG. 2 is an image of pyramid-shaped micro-spicule particles prepared by adding 0.02% weight ratio (FIG. 2a), 0.1% weight ratio (FIG. 2b), 0.5% weight ratio (FIG. 2c) of ethylhexylglycerin to the water-soluble micro-spicule particle manufacturing solution.
FIG. 3a is an image of a diamond-like micro-spicule particles prepared without adding alkylated glycerin to the water-soluble micro-spicule particles manufacturing solution.
FIG. 3b is an image of the diamond-shaped micro-spicule particles prepared by adding 0.03% weight ratio of ethylhexylglycerin to the water-soluble micro-spicule particles manufacturing solution.
FIG. 4 is an image of a diamond-shaped micro-spicule particles prepared by adding 0.02% weight ratio (FIG. 4a), 0.1% weight ratio (FIG. 4b), 0.5% weight ratio (FIG. 4c) of ethylhexylglycerin to the water-soluble micro-spicule particles.
FIG. 5 is an image of pyramid-shaped micro-spicule particles prepared by adding ethanol to a water-soluble micro-spicule particle manufacturing solution of the comparative Example 3.

### DETAILED DESCRIPTION OF PREFERRED EMBODIMENTS

### Description of Drawings

Hereinafter, with reference to the accompanying drawings, the present invention will describe the composition for controlling the shape of the micro-spicule particles, the micro-spicule particles made of a water-soluble material, and its method to producing the same.

The drawings described herein are provided as examples in order to fully transfer the spirit of the invention to those who skilled in the same art. Therefore, the present invention is not limited to the drawings presented, but also is to be embodied in other forms, and the drawings may be exaggerated to demonstrate the spirit of the present invention.

Unless otherwise defined in the technical and scientific terms used herein, it has the meaning commonly understood by one of ordinary skill in the art to which this invention belongs, descriptions of well-known functions and configurations that may unnecessarily obscure the subject matter of the present invention will be omitted in the following description and the accompanying drawings.

As used herein, the singular forms "a", "an" and "the" are intended to include the plural forms as well, unless the specification indicates otherwise.

As used herein, unless stated otherwise, unit of% means weight% unless otherwise defined.

### Mode for invention

The micro-spicule particles according to the present invention include a micro-spicule with four or five pointed vertices that are not fixed to a substrate, wherein the micro-spicule is water-dissolvable and mixed with the non-aqueous media, wherein the micro-spicule comprises more than 80wt% of hyaluronic acid; and a micro-spicule with four or five pointed vertices which comprises more than 80wt% of hyaluronic acid and further comprises alkylated glycerin compounds, and in some cases, may further include materials useful for skin.

According to the present invention, because the micro-spicule particles include an alkylated glycerin compound, and in one detailed example, as the hyaluronic acid aqueous solution containing an alkylated glycerin compound is loaded onto a multiple pattern of micro-spicule form engraved mold, air-dried up, and then tapped off, it is possible to effectively produce a sharp micro-spicule particles shape without collapsing the tip side of micro-spicule.

In addition, the present invention implements the advantageous effect of controlling the shape of the micro-spicule particles by including an alkylated glycerin compound when the micro-spicule particles are prepared.

The advantageous effect of stated above is due to the use of an alkylated glycerin compound. If the alkylated glycerin compound is not used, for example, if glycerin or alcohol is used instead of using alkylated glycerin compound, is not implemented.

The alkylated glycerin compound of stated above is preferably substituted with one or two hydroxyl groups among three hydroxyl groups of the alkylated glycerin which are substituted or non-substituted with alkyl (C4-C20) oxy group. As a preferred and specific example, ethylhexylglycerine can be considered as the alkylated glycerin compound.

Micro-spicule particles according to the present invention can be prepared through an aqueous solution comprising an alkylated glycerin compound and hyaluronic acid, wherein the aqueous solution may comprise 0.001% by weight or higher concentration of the alkylated glycerin compound.

The micro-spicule particles produced by the present invention can adjust the shape of the particles needle-like while maintaining the sharp shape.

More preferably, the aqueous solution contains at least 0.1% of an alkylated glycerin compound. The upper limit of the alkylated glycerin compound is not particularly limited as long as the particles can maintain its desired form.

For example, 5%, specifically 1%, and more specifically 0.5% can be included.

In one example of the present invention, the content of hyaluronic acid in the aqueous solution may be acceptable if the tip of micro-spicule can be molded into a suitable shape.

For example, the aqueous solution may contain 15 to 65% by weight of hydrolyzed hyaluronic acid. However, this is only described as a preferred example, and the present invention is not limited thereto.

In one example of the present invention, the content of water in the aqueous solution may acceptable if the tip of micro-spicule can be molded into a suitable shape. For example, 30 to 80% by weight may comprise water. However, this is only described as a preferred example, and the present invention is not limited thereto.

In another preferred example, when the composition ratio of each component in the aqueous solution is described, in order to demonstrate the above-described effect well, the weight ratio of the hyaluronic acid, the alkylated glycerin compound, and the water may be 1: 0.001∼1:1∼4.

The size of the micro-spicule particles according to the present invention may be acceptable if transdermal delivery is performed effectively, and may be acceptable, for example, 10 to 1,000 µm, specifically 100 to 500 µm.

However, this is only described as a preferred example, and the present invention is not limited thereto.

The shape of the microscopic particles according to the present invention is not particularly limited, but the shape of micro-spicule particles by composition of the present invention include all three-dimensional micro-spicule particles having vertices such as trigonal or tetragonal shapes.

In the case of these micro-spicule particles, when the particles are molded by the composition of the present invention, the shape of the vertices does not be collapsed or become dull, and the micro-spicule particles having a very accurate shape of the vertices are to be obtained.

In addition, their morphological stability may be further increased. Namely, the shape of the micro-spicule particles according to the present invention may be any shape that includes a pointed vertex.

Specific and preferred examples include n-gonal particles. The n may be 3 or higher, but the upper limit value is not greatly limited.

In one example, the shape of the micro-spicule particles may have a trigonal shape or a tetragonal shape in which the bottom surfaces are triangle or quadrangle.

The water-soluble micro-spicule particles according to the present invention have an advantageous effect of delivering the skin useful substances transdermally by further comprising the skin useful substances.

The skin-useful substances stated above may be used in a variety of techniques known in the art, such as cosmetics, medical technology, pharmaceutical technology for the purpose of preventing skin aging, skin moisturization, skin anti-oxidation, and the like.

They may be added in the aqueous solution with hyaluronic acid or together with hyaluronic acid and alkylated glycerin in the preparation process. They also may be used as a mixture of micro-spicule particle combination.

According to the present invention, the water-soluble micro-spicule particles can be applied or used for cosmetic and pharmaceutical use.

According to the present invention, the water-soluble micro-spicule particles have an advantageous effect of delivering the active ingredient transdermally even in a general cosmetic or drug topical formulation.

Hereinafter, the present invention will be described in detail with exemplary embodiments, but these are for explaining the present invention in more detail, and the scope of the present invention is not limited by the following exemplary embodiments.

### EXAMPLES

### Exemplary Embodiment 1. Preparation of Pyramidal Shape Micro-spicule Particles

Ethyl hexyl glycerin was added to the aqueous solution mixed with 2 g of hyaluronic acid and 4 g of water at a weight ratio of 0.02%, 0.03%, 0.1%, and 0.5%, respectively, to prepare a mixed solution for preparing water-soluble micro-spicule particles.

The prepared mixed solution was loaded onto a mold in which the pyramidal shape pattern was engraved, air-dried up, and then the dried micro-spicule particles were separated by tapping to prepare water-soluble pyramidal micro-spicule particles.

### Comparative Example 1.

The procedure is same as that described in Exemplary Embodiment 1, except that no ethyl hexyl glycerin is used.

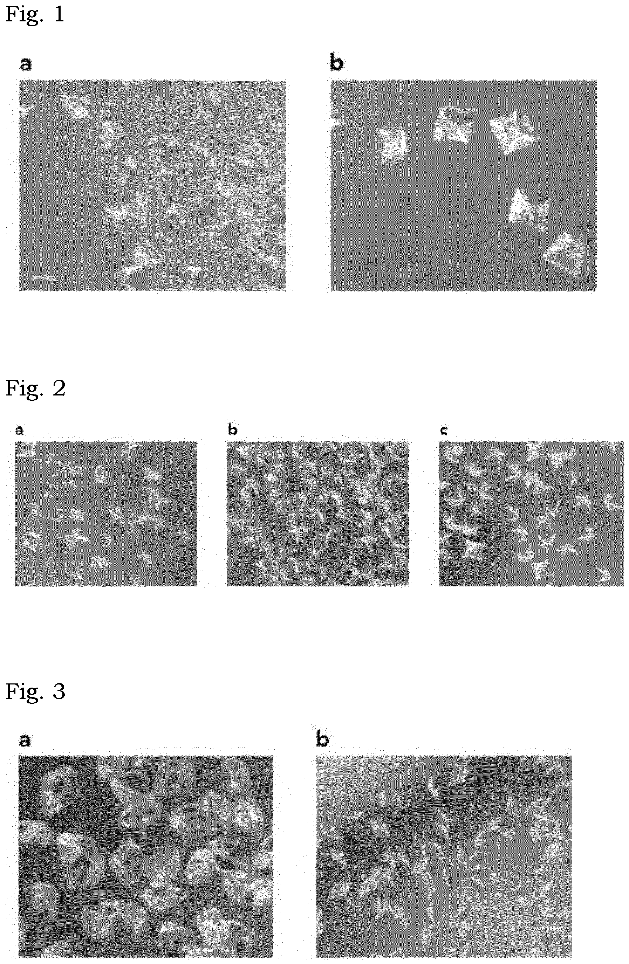

FIG. 1a is an image of pyramidal shape micro-spicule particles prepared according to Comparative Example 1 in which ethylhexylglycerin is not used.

FIG. 1b is an image of pyramidal shape micro-spicule particles prepared with 0.03% by weight of ethylhexylglycerine.

In the Exemplary Embodiment 1 of FIG. 1b, the micro-spicule particles to which ethylhexylglycerin is added can be confirmed that the tips of micro-spicule are kept sharp and not collapsed, whereas in the Comparative Example 1 of FIG. 1a, the tips are collapsed and blunt end can be seen.

FIG. 2 is an image of micro-spicule particles in the Exemplary Embodiment 1 prepared by adding ethylhexylglycerin in 0.02% weight ratio (FIG. 2a), 0.1% weight ratio (FIG. 2b), and 0.5% weight ratio (FIG. 2c).

As seen in FIG. 2 it is confirmed that the bend occurs at the inclined plane of the pyramidal shape micro-spicule in accordance with the increased concentration of ethylhexylglycerin, the angle of the vertex is also changed, in particular sharpen shape is observed as the ethylhexylglycerin concentration increases.

In addition, not only the shape change of the micro-spicule particles but also the porosity change of the micro-spicule particles are observed.

### Exemplary Embodiment 2. Preparation of Diamond-shaped Micro-spicule Particles

In Example 1, except that a diamond-shaped mold was used instead of a pyramid-shaped mold, the same procedure as in Example 1 was performed to prepare diamond-shaped micro-spicule particles formed of a water-soluble material.

### Comparative Example 2.

Using the diamond-shaped mold as described in the Exemplary Embodiment 2, the procedure is the same as that described in the Exemplary Embodiment 1, except that no ethylhexylglycerin is used.

FIG. 3a is an image of diamond-shaped micro-spicule particles prepared by the procedures with no addition of ethylhexylglycerine according to the Comparative Example 2.

FIG. 3b is an image of pyramid-shaped micro-spicule particles prepared by adding 0.03% ethylhexylglycerine. In the Exemplary Embodiment of FIG. 3b, the micro-spicule particles to which ethylhexylglycerin is added can be confirmed that the tips of micro-spicule are kept sharp and not collapsed, whereas in the Comparative Example 2 of FIG. 3a, the tips of micro-spicule are collapsed.

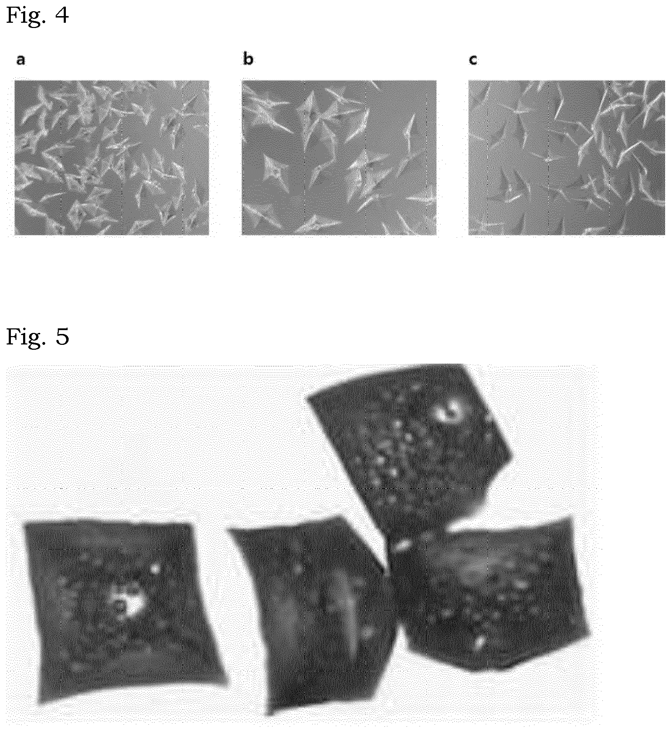

FIG. 4 shows three images of diamond-shaped micro-spicule particles of the Exemplary Embodiment 2 prepared by adding ethylhexylglycerin in 0.02% weight ratio (FIG. 4a), 0.1% weight ratio (FIG. 4b), and 0.5% weight ratio (FIG. 4c).

As seen in the FIG. 4, it is confirmed that the sharper micro-spicule is prepared in accordance with the increased concentration of ethylhexylglycerine, the angle of the vertex is also changed, as the ethylhexylglycerine concentration increases.

In addition, not only the shape change of the micro-spicule particles but also the porosity change of the micro-spicule particles are observed

### Comparative Example 3.

Except that ethanol is used instead of ethylhexylglycerin, the micro-spicule particles are prepared in the same manner as described in the Exemplary Embodiment 1.

As shown in FIG. 5, when the micro-spicule particles prepared by the procedures described in the Comparative Example 3 were checked, collapsed or blunt tips are observed. It is confirmed that too many pores are generated in the micro-spicule particles to use.

### Comparative Example 4.

Except that glycerin is used instead of ethylhexylglycerin, micro-spicule particles are prepared in the same manner as described in the Exemplary Embodiment 1.

Observing the micro-spicule particles prepared by the procedures described in the Comparative Example 4, not only the tip is collapsed as compared with that in the Exemplary Embodiment 1, but also the pores size is relatively large and its number is abundant, it is thus confirmed that there is no significant difference between micro-spicule with glycerin and micro-spicule with ethanol shown in FIG. 5 in the Comparative Example 3.

## Claims

1. A micro-spicule for percutaneous delivery of a dermal cosmetic material, wherein the micro-spicule comprises a tetrahedral or pyramidal body with four or five pointed vertices that are not fixed to a substrate, wherein the micro-spicule is water-dissolvable and mixed with the non-aqueous media, wherein the micro-spicule comprises more than 80wt% of hyaluronic acid.

2. The micro-spicule of claim 1, wherein the micro-spicule further comprises alkylated glycerin.

3. The micro-spicule of claim 2, wherein the alkylated glycerin is substituted with C₄-C₂₀ alkoxy group at one (1) or two (2) of the three (3) hydroxylic group of the glycerin.

4. The micro-spicule of claim 3, wherein the alkylated glycerin is ethyl hexyl glycerin.

5. The micro-spicule of claim 4, which is prepared through molding process of water solution of hyaluronic acid which comprises 0.001 to 1 wt% of ethyl hexyl glycerin.

6. A process for preparation of micro-spicule, which comprises a step of molding aqueous mixture which comprises hyaluronic acid, alkylated glycerin and water, and a step for drying the molded mixture.

7. The process for preparation of micro-spicule according to claim 6, which comprises a step of preparing aqueous mixture which comprises hyaluronic acid, alkylated glycerin and water, a step of filling a mold with the aqueous mixture, and a step for drying the molded aqueous mixture.
